# EUROPEAN PATENT APPLICATION

(11) **EP 1 445 333 A2**
(43) Date of publication of application: **11.08.2004**
(21) Application number: 04290341.9
(22) Date of filing: 10.02.2004
(51) Int. Cl.: C12Q 1/68

(54) **Screening of a novel hepatic syndrome and its uses**

(30) Priority: 10.02.2003 US 360705
(71) Applicant: ASSISTANCE PUBLIQUE, HOPITAUX DE PARIS, 75004 Paris (FR)
(72) Inventor: Poupon, Raoul, 75013 Paris (FR); Hermelin, Brigitte, 78000 Versailles (FR); Rosmorduc, Olivier, 75011 Paris (FR)
(74) Representative: Vialle-Presles, Marie José

(57) **Abstract**

Screening of a hepatic syndrome occurring in young adults and associating (i) intrahepatic hyperechoic foci with or without intrahepatic sludge or microlithiasis and further on a cholesterol microcholelithiasis, intrahepatic cholestasis and (ii) one or more mutations of the *MDR3* gene (point mutations and/or SNPs).

## Description

The present invention relates to the screening of a hepatic syndrome occurring in young adults and associating (i) intrahepatic hyperechoic foci with or without intrahepatic sludge or microlithiasis and further on a cholesterol microcholelithiasis, intrahepatic cholestasis and (ii) one or more mutations of the *MDR3* gene (point mutations and/or SNPs).

Cholesterol cholelithiasis is characterized by the presence of calculi of cholesterol or of calcium bilirubinate. Cholesterol lithiasis is the most common form of cholelithiasis (80%). It is characterized by the formation of calculi of cholesterol in the gall bladder, due to an excess of bile cholesterol compared to solubilizing molecules, namely bile acids and phospholipids. Its prevalence is estimated, taking all ages into account, to be 10 to 20% of the population in industrialized countries. It is more prevalent in women and increases considerably with age (40% in women over the age of 60). Gall bladder lithiasis can be a source of complications: cholecystitis, cancer of the gall bladder, migration of calculi in the common bile duct, a cause of angiocholitis and of pancreatitis. Treatment for this disease is based mainly on surgery: cholecystectomy by laparotomy or celioscopy, biliary drainage with peroperative or perendoscopic extraction of the common bile duct calculi. Medical treatment, which consists of administering chenodeoxycholic acid and/or ursodeoxycholic acid, is reserved for forms of non-complicated cholesterol cholelithiasis of the gall bladder.

Chronic biliary cholestasis is characterized by a deficiency in passage of the bile from the liver to the extrahepatic bile ducts and the intestine. The causes and physiopathology are varied (Poupon et al., 2000, *J. Hepatol.,* 32, 129-140). Most chronic biliary cholestasis can progress to biliary cirrhosis and hepatocellular insufficiency requiring a liver transplant.

In children for example, cholestasis is observed in biliary atresia, in Alagille syndrome or in progressive familial intrahepatic cholestasis (PFIC). In general, genetic abnormalities are observed in these various diseases.

Familial intrahepatic cholestasis (PFIC) are infantile recessive diseases which are characterized by intermittent jaundice, severe cholestasis and cirrhosis, with a fatal outcome during the first ten years of life.

These diseases manifest themselves in several clinical forms, two of which are determined by mutations present on genes encoding ABC transporters. Byler's disease, or progressive familial intrahepatic cholestasis type 1, which is characterized by normal cholesteremia and normal serum γ-glutamyl transferase activity, was the first described. It is caused by mutation of the *FIC1* gene, a type V ATPase involved in phospholipid transport from the outer wall to the inner wall of various cell membranes. More specifically, PFIC type 1 is characterized by recurrent episodes of jaundice, severe pruritis, normal γ-GT activity and normal cholesterol levels, high concentrations of bile acids in the serum and low levels of bile acids in the bile. The PFIC1 locus was found in chromosome 18q21-q22; five mutations were identified: a deletion, a deleted exon and three missense mutations; these missense mutations concern domains which are highly conserved in type V ATPases (E. Jacquemin et al., J. Hepatol., 1999, 31, 377-381).

Familial cholestasis type 2 exhibits the same clinical signs as in Byler's disease, but it is caused by mutations in the *FIC2* gene, which encodes the BSEP *(Bile Salt Export Pump)* transporter. This gene is the homolog of the murine *SPGP* gene, the product of which, expressed exclusively in hepatocytes, is involved in bile salt transport. PFIC type 2 is characterized by a severe pruritis, normal γ-GT activity and a normal cholesterol level in the serum, high concentrations of bile acids in the serum and very low levels of bile acids in the bile. The PIFC2 locus was found in chromosome 2q24: 10 mutations were identified, including deletions and missense mutations involving important domains of the BSEP transporter (E. Jacquemin et al., J. Hepatol., 1999, 31, 377-381).

Individuals suffering from progressive familial intrahepatic. cholestasis type 3 (PFIC3) have mutations in the *MDR3* gene. PFIC type 3 differs from the other PFICs in particular by the fact that it has a high serum γ-glutamyl transferase activity. The MDR *(Multi Drug Resistance)* family of ABC transporters is a multigene family of homologous proteins, some of which (MDR3) are not involved in drug resistance, and are phosphatidylcholine transporters. The *MDR3* gene, revealed in 1993, is a phospholipid translocator. It is expressed in hepatocytes and provides phosphatidylcholine translocation, thus leading to secretion of this phospholipid into the bile.

Mice in which the gene equivalent to *MDR3* has been knocked out, *mdr 2 (-*/*-)*, exhibit a deficiency secretion of phospholipids into the bile, which causes physiological modifications close to those patients suffering from progressive familial intrahepatic cholestasis type 3. Two patients suffering from this disease and carrying mutations on both *MDR3* alleles have recently been described; the histopathological profile of the two patients is similar to that observed in the *mdr 2 (-*/*-)* mice (J. Marleen L. de Vree et al., PNAS, 1998, 95, 1, 282-287). More specifically, at the histological level, portal fibrosis, proliferation in the bile ducts and the presence of an inflammatory infiltrate are observed. However, no cholesterol microcholelithiasis is observed in these patients suffering from PFIC3 or in the *mdr 2 (-*/*-)* mice. At the gene level, in the first patient, a homozygous 7 pb deletion was observed, beginning at amino acid 132, leading to a reading frame shift and introducing a stop codon, 29 codons downstream; the second patient is homozygous for a nonsense mutation in codon 957 (C/T) which introduces a stop codon (TGA): this mutation deletes the *TaqI* restriction site. In such a pathological condition, truncated, nonactive proteins, missing at least one ABC motif, are expressed. Furthermore, additional nonsense mutations and missense mutations, associated with very low levels of bile phospholipids, have also been observed.

Cholestasis appearing only during pregnancy have also been observed in patients exhibiting a heterozygous mutation of the *MDR3* gene; in such cases, no microlithiasis has been observed (E. Jacquemin et al., The Lancet, 1999, 353, 210-211; Dixon et al., Human Molecular Genetics, 2000, 9, 1209-1217).

More specifically, in these patients, the following is observed:
- either a heterozygous deletion of a nucleotide (1712deIT) beginning at amino acid 571, leading to a reading frame shift introducing a stop codon, 15 codons downstream: this mutation, which leads to the expression of a truncated, nonactive protein, was detected in pregnant women from a family suffering from PFIC3 (Jacquemin et al., The Lancet, 1999, mentioned above);
- or a missense mutation in exon 14 (A546D): this mutation was detected in pregnant women with no family history of PFIC (Dixon et al., mentioned above).

The inventors have found (see WO 02/12556) that, in young adults (20-40 years old), pathological conditions exist which differ from PFIC type 3, which are triggered under situations such as pregnancy, the taking of sex hormones, sustained fasting, sustained parenteral nutrition, and surgical interventions, or by hepatotoxic cofactors (obesity, diabetes, the taking of medicines liable to modify the composition of bile: fibrates or diuretics, in particular), and in which the following are observed in combination:
- cholesterol microcholelithiasis, intrahepatic cholestasis and
- at least one point mutation of the *MDR3* gene located in exon 6 and/or in exon 9 and/or in exon 12; more specifically the detected mutations were positioned as follows: a mutation in exon 6 at nucleotide 523, a mutation in exon 9 at nucleotide 959 and a mutation in exon 12 at nucleotide 1327.

More specifically, the detected point mutations were as follows:
- a heterozygous missense mutation in exon 6 at nucleotide 523: A/G (ACG/GCG), leading to the amino acid mutation T175V; this amino acid is located in a conserved amino acid sequence required for the ATPase activity of the MDR3 protein,
- a homozygous missense mutation in exon 9 at nucleotide 959: C/T(TCC/TTC), leading to the amino acid mutation S320F, which is located at the end of transmembrane domain 5 (TM5), and
- a heterozygous mutation in exon 12: insertion of an adenine at nucleotide 1327 (1327insA) in the first nucleotide-binding domain (NBD1); this mutation causes a reading frame shift at codon 443 and the appearance of a stop codon (nt 1339-1341), leading to the expression of a 446 amino acid truncated protein.

Even though the inventors have already shown that the hereabove mentioned point mutations may help to provide a test for screening a new syndrome defined by a decrease in the concentration of bile phospholipids and the presence of cholesterol crystals sensitive to treatment with UDCA, combined with a point mutation of the *MDR3* gene, they have now find that in view of a more accurate screening of said syndrome, it is preferable to detect further on other point mutations which may be found in other positions than the ones disclosed in WO 02/12556 and also to detect single-nucleotide polymorphisms (SNPs), which may also be found in this syndrome, with an abnormal frequency.

Indeed, the onset of this syndrome is related to the appearance, before microlithiasis, of intrahepatic hyperechoic foci with or without intrahepatic sludge.

In this syndrome, as specified in WO 02/12556, a deficiency in bile phospholipids is observed (abnormality of bile phospholipid secretion), limiting the solubilization of cholesterol and leading to the precipitation thereof in the form of microcalculi *in situ* within the small bile ducts and, in extreme forms, within the entire biliary tree.

These microcalculi, which can be detected through examination of the liver by ultrasonography, exhibit the following characteristics:
- they are observed in the form of multiple calculi which are small in size (<2 mm), present in the intrahepatic bile ducts,
- they are sensitive to treatment with ursodeoxycholic acid (UDCA); this treatment makes it possible to obtain rapid disappearance of the symptoms, unlike cholecystectomy, which leads to a recurrence of the disease. This recurrence can, however, be prevented by administering UDCA, and
- they differ from gall bladder calculi by their small size, their location and their sensitivity to treatment with UDCA.

However, it emerges from the new studies of the Inventors that this syndrome is not only charaterized by a decrease in the concentration of bile phospholipids, and the presence of cholesterol crystals sensitive to treatment with UDCA, combined with a point mutation of the *MDR3* gene as those described in WO 02/12556, but may also involve other point mutations and/or SNPs observed with an abnormal frequency. This syndrome is now referred to as Low Phospholipid Associated Cholelithiasis (LPAC).

The screening and the prevention of this pathological condition, which is different from PFIC type 3, in individuals having personal or family history of gall bladder lithiasis or of unexplained hepatic ailments (biological abnormalities relating to tests for cholestasis: increase in serum activity of γ-glutamyltranspeptidases, transaminases) are crucial. Specifically, 10 to 20% of gravid cholestasis might, in fact, correspond to this syndrome, and its morbidity, including repeat abortions, might thus be avoided in the case of more accurate screening and effective prevention.

For this reason, the inventors gave themselves the aim of providing a complementary test for screening this pathological conditions, which differs from progressive familial intrahepatic cholestasis type 3 (PFIC3) both clinically and biochemically, in such a way as to prevent its harmful effects, in particular in women of childbearing condition.

A subject-matter of the present invention is therefore a method for the screening of a hepatic syndrome comprising at least the detection, from a sample of nucleic acid extracted from peripheral blood mononuclear cells, of at least one heterozygous mutation of the *MDR3* gene and/or of a homozygous mutation which does not destroy the expression of the protein expressed by said gene with phosphatidylcholine transporter activity, in adult individuals associating cholesterol microcholelithiasis and intrahepatic cholestasis, characterized in that said MDR3 gene mutation is located in at least one of the following exons: 4, 5, 6, 8, 9, 10, 14, 15, 16, 17, 18, 19, 23 and/or 26 at the following nucleotide positions:
- a mutation in exon 4 at nucleotide 175,
- a mutation in exon 6 at nucleotide 495 or 504, or a a heterozygous missense mutation in exon 6 at nucleotide 523:T/C (TCG/CCG), leading to the amino acid mutation T175A; this amino acid is located in a conserved amino acid sequence required for the ATPase activity of the MDR3 protein,
- a mutation in exon 8 at nucleotide 711,
- a mutation in exon 9 at nucleotide 902,
- a mutation in exon 10 at nucleotides 1007-1015 (insertion or deletion)
- a mutation in exon 14 at nucleotide 1584,
- a mutation in exon 15 at nucleotide 1772,
- a mutation in exon 16 at nucleotide 1954,
- a mutation in exon 17 at nucleotide 1973,
- a mutation in exon 18 at nucleotides 2270-2273 (insertion)
- a mutation in exon 19 at nucleotide 2363,
- a mutation in exon 23 at nucleotide 2800 and
- a mutation in exon 26 at nucleotide 3481.

For the purposes of the present invention, the expression "mutation which does not destroy the expression of the protein having phosphatidylcholine transporter activity" is intended to mean both mutations (point mutation and/or SNPs with abnormal frequency) which lead to the expression of a protein having residual phosphatidyl transporter activity and mutations which decrease the level of expression of the normal protein (mutation in the promoter for example). These various mutations do not induce any hepatobiliary symptoms in children, but the appearance, in young adults, of symptoms associating cholesterol microcholelithiasis and intrahepatic cholestatis, induced by additional factors linked to the host and/or to the environment.

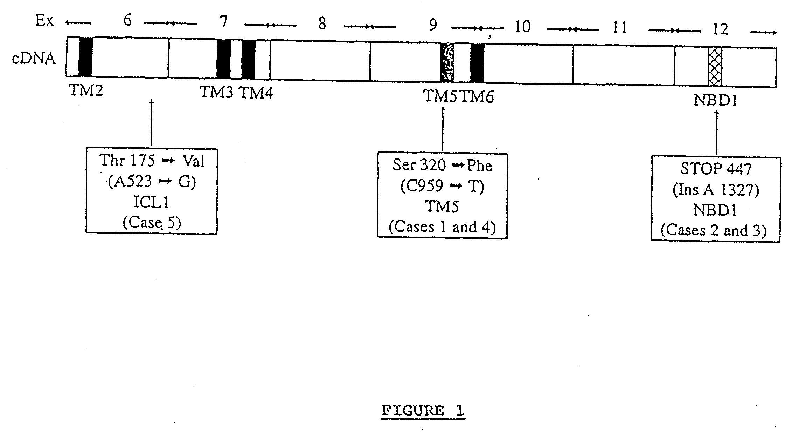

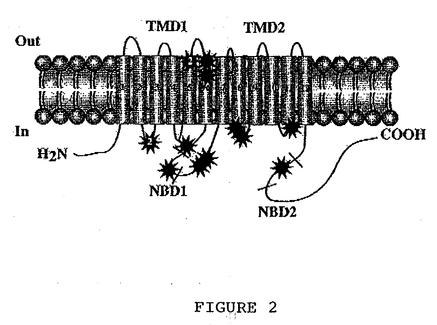

More specifically said mutation is preferably located in exons 9, 10, 14, 15, 17 or 18; indeed, as it emerges from the obtained data, most mutations are localized in the central part of the molecule, close to nucleotide binding domain 1 (NBD1), or in adjacent transmembrane domains and intracellular loops (see figures 1 and 2). More precisely, 80% of mutations are situated in regions encoded by exons 9 to 18, which correspond approximately to 38% of the encoding region (TM5 and 6; 3^{rd} intracellular loop including NBD1; TM7 and 8).

All the detected mutations (point mutations or other type of mutations such as SNPs) are significantly related to LPAC, when, based on a multivariate analysis (see example 1), able to define a clinical score, said score being ≥ 2 (1 point if age at the onset of symptoms was below 40 years, 1 point for recurrence after cholecystectomy and 1 point for the presence of intrahepatic spots).

More specifically, said method comprises the detection of a MDR3 gene mutation, summed up (in italic) in the following Tables I and II, which include the different possible mutations to be screened in LPAC.

**Table I:**

| MDR3 gene point mutations in patient with LPAC syndrome | | | | |
|---|---|---|---|---|
| Gene Position | Location and nucleotide change | **Peptide change** | **Protein domain** | **Status** |
| 6 | *495T→A* *523T→C* | Phe 165 Ile Thr 175 Ala | 1^{st} intracellular loop between TMA-TM3 | Hetero Hetero |
| 9 | *902T→C* 959C→T | Met 301 Thr Ser 320 Phe | TM5 | Hetero Homo |
| 10 | *1007-1015insT* *1007-1015delT* | 355 stop 341 stop | TM6 TM6 | Hetero Hetero |
| 12 | 1327insA | 447 stop | Close to NBD1₁ | Hetero |
| 14 | *1584G→C* | Glu 528 Asp | Close to NBD1₁ | Hetero |
| 15 | *1772T→A* | Leu 591 Gln | 3^{rd} intracellular loop | Homo |
| 17 | *1973G→A* | Try 658 Stop | 3^{rd} intracellular loop linker domain | Hetero |
| 18 | *2270-2273insT* | 793 Stop | 4^{th} intracellular loop between TM8-TM9 | Hetero |
| 19 | *2363G→T* | Arg 788 Glu | 4^{th} intracellular loop between TM8-TM9 | Hetero |
| 23 | *2800G→T* | Ala 934 Thr | 5^{th} intracellular loop between TM10-TM11 | Homo |
| 26 | *3481C→T* | Pro 1161 Se | Close to NBD2 | Hetero |
| Note : The A of ATG of the initiator Met codon was denoted as "nucleotide +1" | | | | |

**Table II:**

| Characterization of *MDR3* gene SNPs and determination of the main allele frequency in patients with and without LPAC syndrome and in control subjects. | | | | | |
|---|---|---|---|---|---|
| SNP Localization | Exon 4 | Exon 5 | Exon 6 | Exon 8 | Exon 16 |
| Nucleotide Change | 175 C→T | 342 T→C | 504 C→T | 711 A→T | 1954 A→G |
| Amino Acid | Leu 59 | Thr 114 | Asn 168 | Ile 237 | Arg 652 Gly |
| Most frequent allele | CTG | ACT | AAC | ATA | AGG |
| Mutation | 21/24 | 24/24 | 16/30 | 21/24 | 23/24 |
| LPAC Phenotype (Score ≥ 2) | (87.5%) | (100%) | (53.3%) | (87.5%) | (95.8%) |
| No mutation | 27/28 | 27/28 | 3/28 | 27/28 | 27/28 |
| LPAC phenotype (Score ≥ 2) | (96.4%) | (96.4%) | (10.7%) | (96.4%) | (96.4%) |
| No mutation | 52/56 | 56/56 | 29/56 | 51/56 | 51/56 |
| No LPAC phenotype (Score < 2) | (92.8%) | (100%) | (51.8%) | (91.1%) | (91.1%) |
| Control subjects | 54/66 | 65/66 | 36/66 | 50/66 | 61/66 |
| | (81.8%) | (98.5%) | (54.5%) | (89.3%) | (92.4%) |
| Bonferroni Adjusted | 0.05 | 0.99 | 0.001 | 0.14 | 0.99 |
| P-value (5 comparisons) | | | | | |

Said Table 11 indicates the most frequent allele to total number of allele ratio for each tested SNPs in the different groups of patients. Nucleotide changes in SNPs are underlined.

Note : The A of ATG of the initiator Met codon was denoted as "nucleotide +1"

Thus five single-nucleotide polymorphisms (SNPs) in the coding region of the MDR3 gene may be identified in LPAC patients besides the point mutations described here above in Table I. In all cases, a high score (as defined here above), i.e. ≥ 2 is obtained.

According to an advantageous embodiment of the method according to the invention, it comprises:
- a first step of amplification of a nucleic acid fragment, in which at least one of said mutations is liable to be observed, from the nucleic acid extracted from peripheral blood mononuclear cells, using at least one primer selected from the group consisting of the primers represented in the herafter Table III under the numbers SEQ NO:15 to SEQ ID NO:66, and
- a second step of detection of the presence of at least one of said mutations, using said amplification fragment(s) obtained.

Said primers may be used in combination with at least two primers described in WO 02/12556, listed as SEQ ID NO:1 to SEQ ID NO:13 in the attached sequence listing.

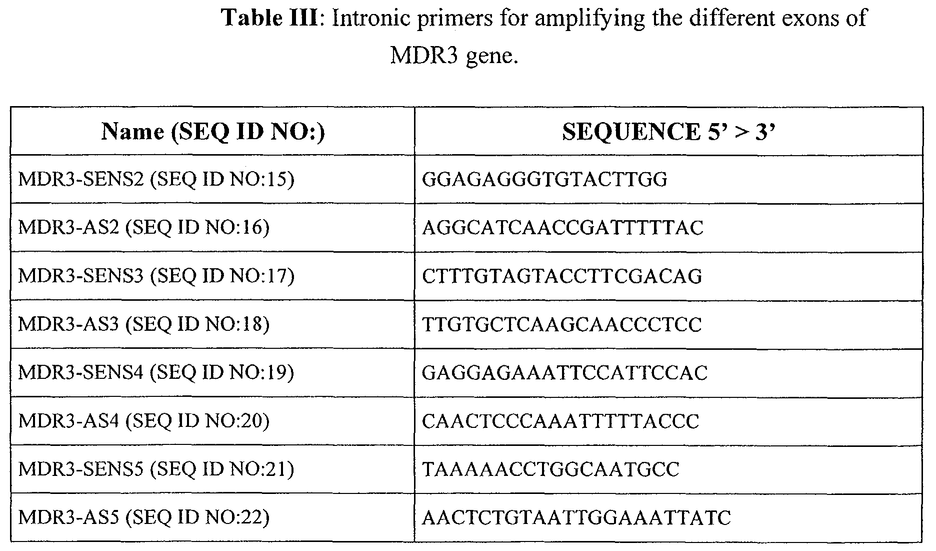

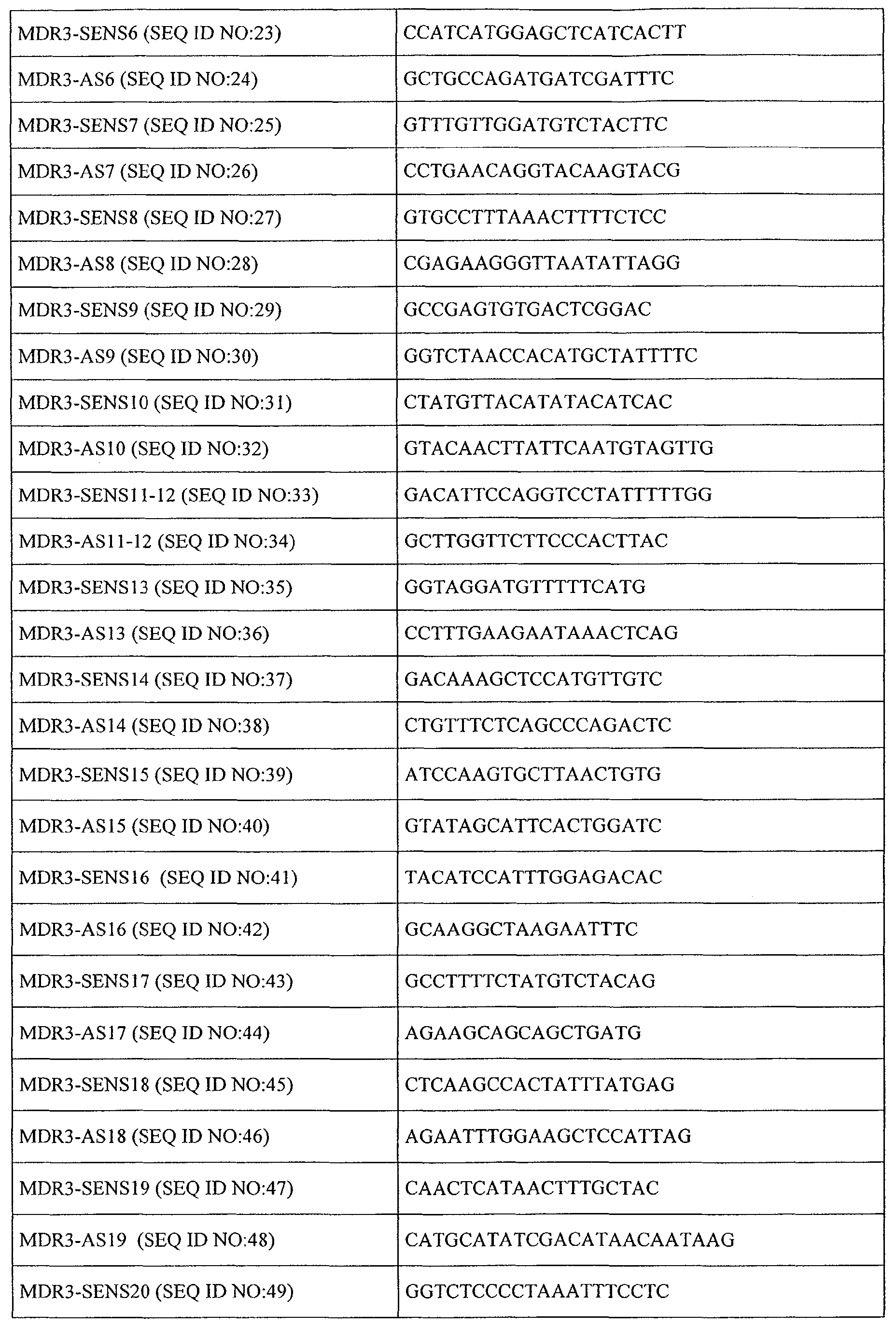

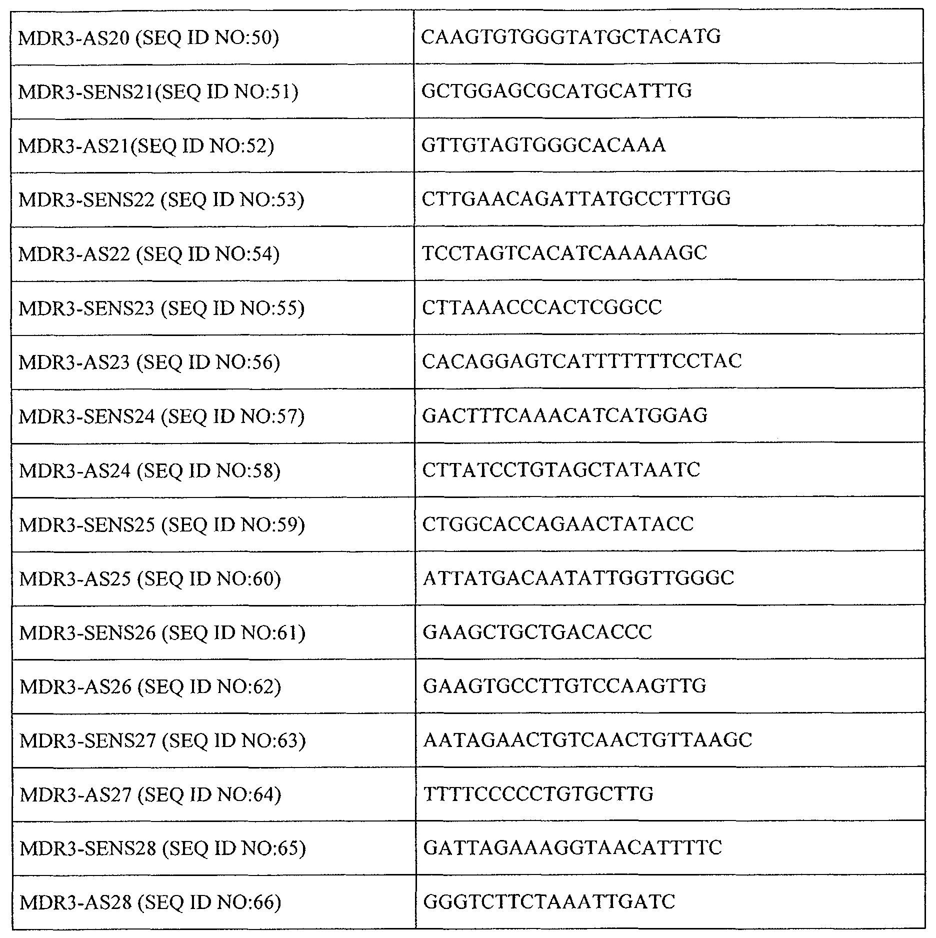

According to the invention, said nucleic acid fragment on which the amplification is performed is selected from the group which consists in mRNA, cDNA and genomic DNA.

According to an advantageous arrangement of this embodiment of the method, the first amplification step consists of direct PCR amplification of at least one of exons 6, 9, 10, 14, 15, 17, 18, 19, 23, and 26 and further of at least one of exons 4, 5, 8 and/or 16 of the genomic DNA with the primers, located in the flanking intronic regions, as specified in the hereabove Table III (SEQ ID NO:15 to 66).

According to yet another advantageous arrangement of this embodiment of the method, the second step of detection of the presence of at least one of said mutations using the amplified fragment is carried out, as appropriate, using one of the following methods:
- sequencing
- enzyme restriction or
- techniques of the PCR/PCR/LDR reaction.

When the mutation results in the appearance or disappearance of a restriction site in the amplified fragment, the mutations are detected by digestion with the enzyme recognizing said restriction site, compared with a control which does not exhibit said mutation.

When the mutation cannot be detected by enzyme digestion, the fragment amplified by PCR is sequenced according to conventional techniques; by way of example, mention may be made of the dideoxynucleotide technique.

When the mutation to be detected corresponds to the insertion or to the deletion of a small sequence, said mutation can be detected using techniques of the PCR/PCR/LDR type, which are based on detecting the ligation of allele-specific primers using a thermostable ligase (Favis et al., *Nature Biotech.,* 2000, 18, 561-564).

Such a method may advantageously be used to evaluate the risk of appearance of a syndrome associating intrahepatic cholestasis and cholesterol microcholelithiasis in families at risk of cholesterol lithiasis and/or having unexplained hepatic biological abnormalities.

When a mutation, as defined above, is detected in individuals at risk, said evaluation method can advantageously also comprise searching for intrahepatic microcrystals of cholesterol, measuring the cholesterol saturation index of the bile, determining the cholesterol/bile phospholipids ratio, assaying bile phospholipids, testing against ursodeoxycholic acid and analysing the clinical relation between said mutation and said hepatic abnormalities by establishing a clinical score based on multivariate analysis on the basis of the following parameters age, recurrence of cholecystectomy and intrahepatic spots.

The diagnosis may be considered to be positive for the abovementioned syndrome when one of the abovementioned mutations is screened, associated with the presence of intrahepatic hyperechoic foci with or without intrahepatic sludge or microlithiasis and further on cholesterol microcholelithiasis, a very low level of bile phospholipids and a cholesterol saturation index of the bile of greater than 1.

Another subject-matter of the present invention is also the use of the various primers as defined above, for the screening of a syndrome associating (i) intrahepatic hyperechoic foci with or without intrahepatic sludge or microlithiasis and further on cholesterol microcholelithiasis, intrahepatic cholestasis and (ii) at least one mutation of the *MDR3* gene, as specified hereabove, optionally associated with at least one mutation as described in WO 02/12556.

More specifically, the invention includes the use of the primers selected from the group consisting of the sequences SEQ ID NO:15 to SEQ ID NO:66, optionally in combination with primers selected from the group consisting of the sequences the primers of SEQ ID NO:1 to SEQ ID NO:13 for the screening of a syndrome associating (i) intrahepatic hyperechoic foci with or without intrahepatic sludge or microlithiasis and further on cholesterol microcholelithiasis, intrahepatic cholestasis and (ii) at least one mutation of the *MDR3* gene.

Another subject-matter of the present invention is also a kit for the screening of a syndrome associating (i) intrahepatic hyperechoic foci with or without intrahepatic sludge or microlithiasis and further on cholesterol microcholelithiasis, intrahepatic cholestasis and (ii) at least one mutation of the *MDR3* gene, characterized in that it comprises at least two primers as defined above, capable of detecting a mutation of the human *MDR3* gene, as defined above.

More specifically, the invention includes a kit for the screening of a syndrome associating (i) intrahepatic hyperechoic foci with or without intrahepatic sludge or microlithiasis and further on cholesterol microcholelithiasis, intrahepatic cholestasis and (ii) at least one mutation of the *MDR3* gene, characterized in that it comprises at least two primers selected from the group consisting of the sequences SEQ ID NO:15 to SEQ ID NO:66, optionally in combination with two primers selected from the group consisting of the sequences SEQ ID NO:1 to SEQ ID NO:13.

Another subject-matter of the present invention is also a kit for evaluating the risk of appearance of a syndrome associating (i) intrahepatic hyperechoic foci with or without intrahepatic sludge or microlithiasis and further on cholesterol microcholelithiasis, intrahepatic cholestasis and (ii) at least one mutation of the *MDR3* gene, in a population at risk, characterized in that it comprises, besides the primers capable of detecting the mutation of the *MDR3* gene as defined above, reagents for assaying cholesterol and bile phospholipids.

More specifically, the invention includes a kit for evaluating the risk of appearance of a syndrome associating (i) intrahepatic hyperechoic foci with or without intrahepatic sludge or microlithiasis and further on cholesterol microcholelithiasis, intrahepatic cholestasis and (ii) at least one mutation of the *MDR3* gene, in a population at risk, characterized in that it comprises, besides two primers selected from the group consisting of the sequences SEQ ID NO:15 to SEQ ID NO:66, optionally in combination with two primers selected from the group consisting of the sequences SEQ ID NO:1 to SEQ ID NO:13, reagents for assaying cholesterol and bile phospholipids.

Preferably, the reagents are enzymatic reagents.

Besides the above arrangements, the invention also comprises other arrangements which will emerge from the following text, which refers to examples of implementation of the present invention and also to the attached drawings in which:
- Figure 1 illustrates the location of the various mutations on the cDNA encoding the MDR3 protein. Exons (Ex) 6 to 12 are represented by white boxes, the black boxes correspond to transmembrane domains (TM) 2 to 6 and the hashed box represents the nucleotide-binding domain (NBD1). The location of the various mutations is represented by arrows. ICL1 corresponds to the intracellular loop of the MDR3 protein, which is essential for the ATPase activity of this protein.
- Figure 2 illustrates the localization of the mutations in the different domains of the MDR3 protein (disease-causing mutations or DCMs). The gray boxes correspond to the transmembrane domains (TMD) of the protein. TMD1 and 2 correspond to the two symmetrical regions containing the TM domains 1-6 and 7-12, respectively. NBD1 and 2 correspond to the nucleotide binding domains and the black stars correspond to the mutations that have been identified in patients presenting LPAC. The numbers in the stars indicate the number of patients having the corresponding mutation.
- Figure 3 illustrates the study profile of example 1.
- Figure 4 illustrates MDR3 gene mutations in LPAC and other human liver diseases: genotype-phenotype relationship.

### Example 1 : Materials and methods

### 1- Characteristics of the patients included in the study

### Patients

32 patients, who were referred specifically for *MDR3* gene analysis because of clinical history compatible with the syndrome that is described hereabove (e.g. having symptomatic cholelithiasis with at least one of the following criteria : age less than 40 years at onset of symptoms, symptomatic cholelithiasis, recurrence after cholecystectomy, intrahepatic hyperechoic foci with a topography compatible with lipid deposits along the luminal surface of the intrahepatic biliary tree with or without intrahepatic sludge or microlithiasis, familial history of cholelithiasis in 1^{st} degree relatives, clinical history of intrahepatic cholestasis of pregnancy), were studied.

A second group of twenty-eight other patients presenting with a classic form of gallstone disease shown by a single episode (or a second episode in one case) of typical biliary pain or cholecystitis that justified cholecystectomy constituted a control group. None of these patients had inflammatory biliary diseases (such as primary biliary cirrhosis or primary sclerosing cholangitis), fibrocystic liver disease (Caroli's disease or congenital hepatic fibrosis), Osler-Weber-Rendu disease, cystic fibrosis, protoporphyria, total parenteral nutrition, obesity (BMI≥ 30 kg/m²), biliary infection, infestation with parasites (including HIV infection) or malabsorption (including ileal resection) diagnosed on the basis of clinical examination and appropriate investigations and none received somatostatin synthetic analogs or hypo-cholesterolemic agent therapy. None of these patients had a medical history of progressive familial intrahepatic cholestasis during childhood and patients suffering from black pigment stones, hemolysis or cirrhosis were also excluded.

A third group comprises 33 consecutive patients without history of cholelithiasis and presenting with chronic cholestasis (primary biliary cirrhosis n = 4, primary sclerosing cholangitis n = 4), diverse acute or chronic liver diseases (chronic HBV or HCV hepatitis, non-alcoholic steatohepatitis (NASH), drug-induced hepatitis) (n=15) or a classic genetic hemochromatosis associated with C282Y homozygous mutation in the *HFE* gene (n=10) constituted a second control group. Based on previous data, the clinical feature characteristic of the syndrome were collected for the 60 patients with cholelithiasis as summed up in the following Table IV.

**Table IV:**

| Patient characteristics | | |
|---|---|---|
| Patients (n) | 60 | |
| Age (years ± SD) | 38.1 ± 14.4 | |
| Gender (M/F) | 15/45 | |
| | (n) | (%) |
| Age at onset of symptoms < 40 years | 37/60 | 62 |
| Symptomatic cholelithiasis | 60/60 | 100 |
| Recurrence after cholecystectomy | 28/60 | 47 |
| Cholecystectomy | 54/60 | 90 |
| Intrahepatic hyperechoic foci ^{*} | 32/60 | 53 |
| Medical history of ICP | 15/46 | 33 |
| Increased serum GGT activity** | 22/60 | 37 |
| Familial history of cholelithiasis | 21/50 | 42 |
| Prophylactic UDCA treatment | 25/39 | 64 |
| UDCA denotes ursodeoxycholic acid. | | |
| ICP denotes intrahepatic cholestasis of pregnancy. | | |

| | | |
|---|---|---|
| * e.g. consisting intrahepatic hyperechoic foci with a topography compatible with lipid deposits along the luminal surface of the intrahepatic biliary tree with or without intrahepatic sludge or microlithiasis. | | |
| ** at the time of genotype determination | | |

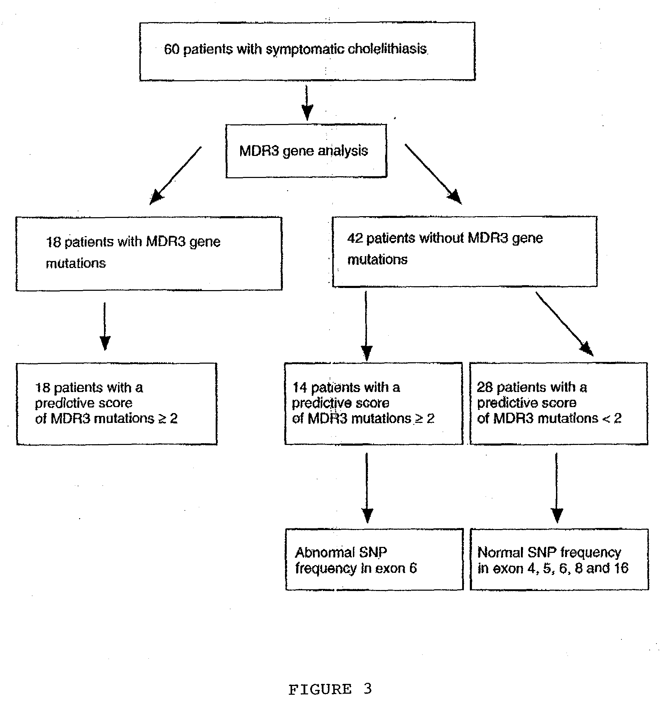

To determine the prevalence of the *MDR3* mutations and single-nucleotide polymorphisms (SNPs), and the clinical factors predictive of these mutations in patients with symptomatic cholelithiasis, the entire *MDR3* sequence in all 93 patients (see Figure 3) was analysed. Written, informed consent was obtained from all participants.

### Mutation screening

Genomic DNA was obtained from peripheral white blood cells using standard procedures. Specific primers to amplify exons and splice junctions, using the Massachusetts Institute of Technology web site (http://www-genome.wi.mit.edu/cgi-bin/primer/primer3-www.cgi) were designed. The sequences of these intronic primers are those described in the here above Table III.

Each PCR reaction contained 200 ng genomic DNA, with each primer at a concentration of 0.4 µM, 0.08 µM deoxynucleoside triphosphates (Pharmacia, Piscataway, N.J.), 1.5 mM magnesium chloride and 1.25 U Taq polymerase. PCR products were purified on sephadex column and sequenced using Big Dye Terminator Chemistry (Applied Biosystems). Identification and localization of *MDR3* gene mutations and SNPs was assessed by sequence comparisons using Phred Phrap Consed Software or Seqscape software (version 1; Applied Biosystems).

### Statistical analysis

Fisher's exact test was used to compare proportions. All tests were at the 5% level, and reported P-values were two-sided. A multivariate, logistic regression model was used to select a set of clinical features predictive of a point mutation at the *MDR3* locus. The multivariate model was selected by minimising the Akaike information criterion, a standard statistical procedure for selecting variables to be included as predictors (Burnham K. et al., Model selection and multi-model interference. NY, Springler Verlag, 2002). When comparing SNPs at the *MDR3* locus, the first species risk was adjusted to the number of comparisons (= number of SNPs) according to the Bonferroni rule. All statistical analyses were performed using the R software (version 1.5.1, http://cran.r-project.org) (Ihaka R. et al., J. Computational and Graphical Statistics, 1996,. 5, 299-314).

### Example 2: Results

### 1. Clinical phenotype associated with MDR3 gene mutations

**Table V:**

| Adjusted odds-ratios (OR) for the presence of a mutation at the *MDR3* locus in patients with cholelithiasis. | | | | |
|---|---|---|---|---|
| Clinical Criteria | OR | CI 95% | P-value | Adjusted OR |
| Familial history of cholelithiasis in 1^{st} degree relatives Increased serum GGT activity at the | 5,4 | [1.2, 29.4] | 0.01 | - |
| | 1.1 | [0.3,4.1] | 1 | - |
| time of genotype determination | 4.9 | [1.1, 24.0] | 0.02 | - |
| History of ICP Intrahepatic hyperchoic foci | 12.4 | [2.4, 126.0] | 0.0005 | 6.1 |
| Recurrence after cholecystectomy | 18.9 | [3.6, 193.7] | <0.0001 | 8.5 |
| Age at onset of symptoms < 40 | 7.8 | [1.5, 77.8] | 0.008 | 3.0 |
| Gender (Male vs Female) | 0.8 | [0.1, 3.4] | 1 | - |

Patients were screened for mutations in the MDR3 gene by using polymerase chain reaction amplification and DNA sequencing of exons 2 to 28 and all splice junctions.

Among the 32 patients suspected of having the syndrome, 18 (56%) presented a point mutation in the *MDR3* gene, whereas none of the 28 patients with a classic form of cholelithiasis and none of the 33 patients without cholelithiasis had mutation in this gene (p < 0,001 and p < 0,0001, respectively). The unadjusted odds-ratios (OR) for the presence of a *MDR3* gene mutation in this population are presented in the entire population of 60 patients with symptomatic cholelithiasis (Table V). Multivariate analysis showed that three independent factors were predictive of a mutation at the *MDR3* locus : a recurrence of symptoms after cholecystectomy (adjusted OR=8.5), intrahepatic hyperechoic foci, intrahepatic sludge, a microlithiasis (adjusted OR=6.1), and age < 40 years (adjusted OR=3.0).

Based on the multivariate analysis, a clinical score indicative of the presence of a *MDR3* mutation was defined as follows: 1 point if age at the onset of symptoms was less than 40 years, 1 point for recurrence after cholecystectomy and 1 point for the presence of intrahepatic spots.

All patients with a point mutation scored higher than 2 (mean ± SE: 2.7 ± 0.5). In contrast, patients without point mutations had scores ranging from 0 to 3 (mean ± SE: 1.2 ± 1.1) and could be divided into two groups (see Figure 3): one group of patients scoring ≥ 2 (n=14) and another group scoring < 2 (n=28). The score was therefore highly sensitive for the presence of a mutation (Se = 100%, CI 95% [85%-100%]) but was not specific in this sample of patients (Sp= 67%, CI 95% [52%-81%]) (Se = Sensitivity; Sp = specificity). Specificity was calculated as the proportion of scores <2 among patients without a mutation, and sensitivity was calculated as the proportion of scores ≥ 2 among individuals with a mutation.

### 2. Identification of MDR3 gene mutation in patients with the syndrome (Table I)

For the purposes of this study, only MDR3 sequence alterations that led to premature truncation of the protein, small deletions, insertions, and missense mutations were considered as potential disease-causing mutations (DCMs).

Patients were screened for mutations in the *MDR3* gene using PCR amplification and DNA sequencing of exons 2 to 28 and all splice junctions. 14 heterozygous and homozygous point mutations were identified among these 18 patients, all of them with a predictive score ≥ 2 (Table I). None of these mutations was detected in a control panel of one hundred and forty chromosomes, thus demonstrating that they did not correspond to polymorphisms and were therefore considered to be DCMs.

These DCMs included three 1bp-insertions and one 1bp-deletion, resulting in a frameshift predicted to cause premature messenger RNA termination, a loss of protein function and ten single-nucleotide substitutions including one null mutation. Affected patients with heterozygous mutations showed 1 bp-insertion, 1 bp-deletion, nonsense mutations or missense mutations, whereas affected patients with homozygous mutations showed only missense mutations. Most mutations (DCMs) were localized as mentioned here above in the central part of the molecule, close to nucleotide binding domain 1 (NBD1), or in adjacent transmembrane domains and intracellular loops (see Figure 2). Eighty percent of mutations were indeed situated in regions encoded by exons 9 to 18, which corresponded approximately to 38% of the encoding region (TM 5 and 6; 3^{rd} intracellular loop including NBD1, TM 7 and 8).

In contrast, no point mutations could be detected in 14 patients with a high score (≥ 2) and 28 patients with a low score (< 2) (see Figure 3). Furthermore, *MDR3* gene sequence analysis in the 33 patients without cholelithiasis showed the presence of two heterozygous missense mutations affecting non-conserved amino acids.

However, because these mutations affected nonconserved amino acids between the human MDR3 gene and its rodent homologs, they were not considered as DCMs. They were localized in the third intracellular loop and in the fourth extracellular loop of the protein (e.g., Arg590Gln and Gly742Ser).

### 3. Identification of MDR3 gene SNPs in patients with cholelithiasis (see Table II)

In contrast to DCMs, which are associated with the susceptibility of a disease and are not detected in controls, SNPs are the most frequent type of genetic variations, are usually not associated with a disease, and are detected in both patients and controls.

To further identify *MDR3* gene defects in patients with a high score but no point mutation, the frequency of *MDR3* gene SNPs in these patients (see Figure 2) was investigated. The previously described Arg652GIy polymorphism was detected with a similar frequency (about 5%) in patients with and without *MDR3* mutations and in control subjects (Jacquemin E. et al., Gastroenterology, 2001, 120, 1448-1458).

Five novel single-nucleotide polymorphisms (SNPs) in the coding region of the *MDR3* gene have also been identified (see Table II here above). The allele frequency of the most frequent variant (e.g. 504 T→C, AAC) was only 10.7% in the group of LPAC syndrome patients with a high score but no *MDR3* point mutations, while it reached 54.5% in control subjects, 51.8% in patients with cholelithiasis and a low score and 53.3% in patients with LPAC syndrome and *MDR3* gene mutations (Bonferroni adjusted P-value < 0.001). Other SNPs were more rare and were detected at similar frequencies in the different patient groups and in control subjects.

The mutation screening described in the invention method was unable to detect major DNA rearragements, and nor did the analysis include the promoter or other potential regulation regions of the gene. Despite these limitations, the unexpected low frequency of the 504 C > T variant (Leu 168 Leu) in the subgroup of patients with LPAC syndrome but without an *MDR3* gene point mutation suggests that some of them may have been hemizygous for this region, so that a large deletion in exon 6 could be implicated in the disease. Such synonymous single-nucleotide polymorphisms located in coding regions, although seemingly translationally silent, could also have a profound influence on alternative splicing and potentially lead to exon skipping or aberrant splicing. Alternatively, defects in other regions of the gene or in other genes may also be involved, and some evidence from animal studies has pointed to Abcb 11 (previously called the bile salt export pump, *BSEP)* or Abcc 2 (previously referred to as multidrug resistance related protein 2, or *MRP2)* as other possible candidate genes underlying susceptibility to cholelithiasis or phospholipid secretion disruption.

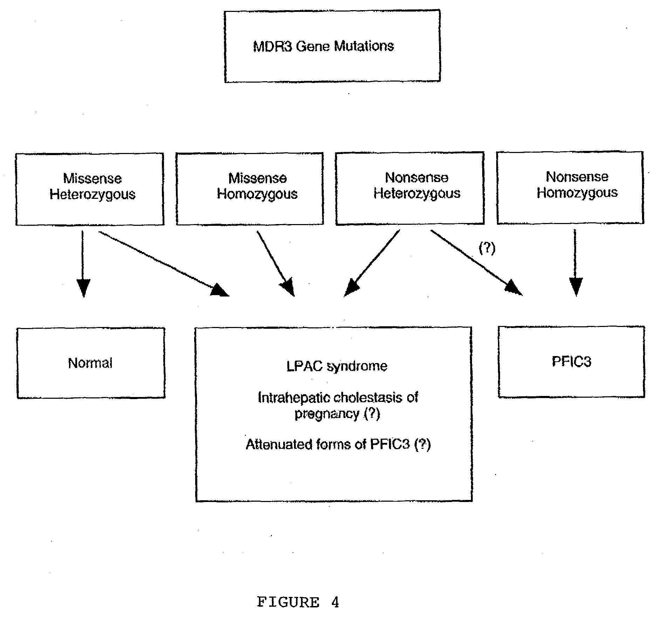

In summary, the results strongly support the role of an *MDR3* gene defect in LPAC syndrome and replace it in the context of *MDR3* gene-associated liver diseases (see figure 4).

## Claims

1. A method for the screening of a hepatic syndrome comprising at least the detection, from a sample of nucleic acid extracted from peripheral blood mononuclear cells, of at least one heterozygous mutation of the *MDR3* gene and/or of a homozygous mutation which does not destroy the expression of the protein expressed by said gene with phosphatidylcholine transporter activity, in adult individuals associating cholesterol microcholelithiasis and intrahepatic cholestasis, **characterized in that** said MDR3 gene mutation is located in at least one of the following exons: 4, 5, 6, 8, 9, 10, 14, 15, 16, 17, 18, 19, 23 and/or 26 at the following nucleotide positions:
- a mutation in exon 4 at nucleotide 175,
- a mutation in exon 6 at nucleotide 495 or 504, or a a heterozygous missense mutation in exon 6 at nucleotide 523:T/C (TCG/CCG), leading to the amino acid mutation T175A; this amino acid is located in a conserved amino acid sequence required for the ATPase activity of the MDR3 protein,
- a mutation in exon 8 at nucleotide 711,
- a mutation in exon 9 at nucleotide 902,
- a mutation in exon 10 at nucleotides 1007-1015 (insertion or deletion)
- a mutation in exon 14 at nucleotide 1584,
- a mutation in exon 15 at nucleotide 1772,
- a mutation in exon 16 at nucleotide 1954,
- a mutation in exon 17 at nucleotide 1973,
- a mutation in exon 18 at nucleotides 2270-2273 (insertion)
- a mutation in exon 19 at nucleotide 2363,
- a mutation in exon 23 at nucleotide 2800 and
- a mutation in exon 26 at nucleotide 3481.

2. The method as claimed in claim 1, **characterized in that** said MDR3 gene mutation is preferably located in exons 9, 10, 14, 15, 17 or 18.

3. The method as claimed in claim 1 or in claim 2, **characterized in that** it comprises:
- a first step of amplification of a nucleic acid fragment, in which at least one of said mutations is liable to be observed, from the nucleic acid extracted from peripheral blood mononuclear cells, using at least one primer selected from the group consisting of the primers having the sequences SEQ ID NO:15 to SEQ ID NO:66, and
- a second step of detection of the presence of at least one of said mutations, using said amplification fragment(s) obtained.

4. The method as claimed in claim 3, wherein the first amplification step consists of direct PCR amplification of at least one of exons 6, 9, 10, 14, 15, 17, 18, 19, 23, and 26 and further of at least one of exons 4, 5, 8 and/or 16 of the genomic DNA with the primers, located in the flanking intronic regions, as specified in SEQ ID NO:15 to 66.

5. The method as claimed in claim 3 **characterized in that** the second step of detection of the presence of at least one of said mutations using the amplified fragment is carried out, as appropriate, using one of the following methods: sequencing, enzyme restriction or techniques of the PCR/PCR/LDR type.

6. The method as claimed in any one of claims 3 to 5 **characterized in that** the detection step further comprises in said first step of amplification the use of at least two primers selected from the group consisting of the sequences SEQ ID NO:1 to SEQ ID NO:13.

7. The use of the method as claimed in claim 1, for evaluating the risk of appearance of a syndrome associating intrahepatic hyperechoic foci with or without intrahepatic sludge or microlithiasis and further on intrahepatic cholestasis and cholesterol microcholelithiasis in families at risk from cholesterol lithiasis and/or having unexplained hepatic biological abnormalities.

8. The use of the primers selected from the group consisting of the sequences SEQ ID NO:15 to SEQ ID NO:66, optionally in combination with primers selected from the group consisting of the sequences the primers of SEQ ID NO:1 to SEQ ID NO:13 for the screening of a syndrome associating (i) intrahepatic hyperechoic foci with or without intrahepatic sludge or microlithiasis and further on cholesterol microcholelithiasis, intrahepatic cholestasis and (ii) at least one mutation of the *MDR3* gene.

9. A kit for the screening of a syndrome associating (i) intrahepatic hyperechoic foci with or without intrahepatic sludge or microlithiasis and further on cholesterol microcholelithiasis, intrahepatic cholestasis and (ii) at least one mutation of the *MDR3* gene, **characterized in that** it comprises at least two primers selected from the group consisting of the sequences SEQ ID NO:15 to SEQ ID NO:66, optionally in combination with two primers selected from the group consisting of the sequences SEQ ID NO:1 to SEQ ID NO: 13.

10. A kit for evaluating the risk of appearance of a syndrome associating (i) intrahepatic hyperechoic foci with or without intrahepatic sludge or microlithiasis and further on cholesterol microcholelithiasis, intrahepatic cholestasis and (ii) at least one mutation of the *MDR3* gene, in a population at risk, **characterized in that** it comprises, besides two primers selected from the group consisting of the sequences SEQ ID NO:15 to SEQ ID NO:66, optionally in combination with two primers selected from the group consisting of the sequences SEQ ID NO:1 to SEQ ID NO:13, reagents for assaying cholesterol and bile phospholipids.


SEQUENCE LISTING

<110> ASSISTANCE PUBLIQUE-HOPITAUX DE PARIS
POUPON, Raoul
HERMELIN, Brigitte
ROSMORDUC, Olivier

<120> SCREENING OF A NOVEL HEPATIC SYNDROME AND ITS USES

<130> BLOcp1020CAS13

<150> US 10/360705
<151> 2003-02-10

<160> 66

<170> PatentIn version 3.1

<210> 1
<211> 21
<212> DNA
<213> Artificial sequence

<220>
<223> Artificial sequence description : PRIMER
<400> 1
ctactcagga ttgggtgctg g 21

<210> 2
<211> 19
<212> DNA
<213> Artificial sequence

<220>
<223> Artificial sequence description : PRIMER

<400>  2
gctagaacat ggctgccag                                                    19

<210>  3

<211>  20

<212>  DNA

<213>  Artificial sequence

<220>

<223>  Artificial sequence description : PRIMER

<400>  3
ccctctcatt tttctggtag                                                   20

<210>  4

<211>  20

<212>  DNA

<213>  Artificial sequence

<220>

<223>  Artificial sequence description : PRIMER

<400>  4
gttaggagaa ctacttactg                                                   20

<210>  5

<211>  20

<212>  DNA

<213>  Artificial sequence

<220>

<223>  Artificial sequence description : PRIMER

<400>  5
ccttacagat cttgaagggc                                                   20

<210>  6

<211>  20

<212>  DNA

<213>  Artificial sequence

<220>

<223>  Artificial sequence description : PRIMER

<400>  6
gcttggttct tcccacttac                                                    20

<210>  7

<211>  23

<212>  DNA

<213>  Artificial sequence

<220>

<223>  Artificial sequence description : PRIMER

<400>  7
cctgccagac acgcgcgagg ttc                                                23

<210>  8

<211>  25

<212>  DNA

<213>  Artificial sequence

<220>

<223>  Artificial sequence description : PRIMER

<400>  8
cttcaagtcc atcggtttcc acatc                                              25

<210>  9

<211>  19

<212>  DNA

<213>  Artificial sequence

<220>

<223>  Artificial sequence description : PRIMER

<400>  9
ccttgtcgct gctaaatcc                                                     19

<210>  10

<211>  21

<212>  DNA

<213>  Artificial sequence

<220>
<223>  Artificial sequence description : PRIMER

<400>  10
ggctcttctg acacatttgt g                                                21

<210>  11
<211>  20
<212>  DNA
<213>  Artificial sequence

<220>
<223>  Artificial sequence description : PRIMER

<400>  11
ggaattggtg acaaggttgg                                                  20

<210>  12
<211>  25
<212>  DNA
<213>  Artificial sequence

<220>
<223>  Artificial sequence description : PRIMER

<400>  12
gctatttcag caaacatttc catgg                                            25

<210>  13
<211>  22
<212>  DNA
<213>  Artificial sequence

<220>
<223>  Artificial sequence description : PRIMER

<400>  13
gctaacgtca agatcttgaa gg                                               22

<210>  14

<211>  18

<212>  DNA

<213>  Artificial sequence

<220>

<223>  Artificial sequence description : PRIMER

<400>  14
gaggccaacg cctatgag                                                    18

<210>  15

<211>  17

<212>  DNA

<213>  Artificial sequence

<220>

<223>  Artificial sequence description : PRIMER

<400>  15
ggagagggtg tacttgg                                                     17

<210>  16

<211>  20

<212>  DNA

<213>  Artificial sequence

<220>

<223>  Artificial sequence description : PRIMER

<400>  16
aggcatcaac cgatttttac                                                  20

<210>  17

<211>  20

<212>  DNA

<213>  Artificial sequence

<220>

<223>  Artificial sequence description : PRIMER

<400>  17
ctttgtagta ccttcgacag                                                  20

<210>  18

<211>  20

<212>  DNA

<213>  Artificial sequence



<220>

<223>  Artificial sequence description : PRIMER

<400>  18
ttgtgctcaa gcaaccctcc                                                    20

<210>  19

<211>  20

<212>  DNA

<213>  Artificial sequence



<220>

<223>  Artificial sequence description : PRIMER

<400>  19
gaggagaaat tccattccac                                                    20

<210>  20

<211>  20

<212>  DNA

<213>  Artificial sequence



<220>

<223>  Artificial sequence description : PRIMER

<400>  20
caactcccaa atttttaccc                                                    20

<210>  21

<211>  18

<212>  DNA

<213>  Artificial sequence



<220>

<223>  Artificial sequence description : PRIMER

<400>  21
taaaaacctg gcaatgcc                                                    18

<210>  22

<211>  22

<212>  DNA

<213>  Artificial sequence

<220>

<223>  Artificial sequence description : PRIMER

<400>  22
aactctgtaa ttggaaatta tc                                               22

<210>  23

<211>  21

<212>  DNA

<213>  Artificial sequence

<220>

<223>  Artificial sequence description : PRIMER

<400>  23
ccatcatgga gctcatcact t                                                21

<210>  24

<211>  20

<212>  DNA

<213>  Artificial sequence

<220>

<223>  Artificial sequence description : PRIMER

<400>  24
gctgccagat gatcgatttc                                                  20

<210>  25

<211>  20

<212>  DNA

<213>  Artificial sequence

<220>

<223>  Artificial sequence description : PRIMER

<400>  25
gtttgttgga tgtctacttc                                                    20

<210>  26

<211>  20

<212>  DNA

<213>  Artificial sequence

<220>

<223>  Artificial sequence description : PRIMER

<400>  26
cctgaacagg tacaagtacg                                                    20

<210>  27

<211>  20

<212>  DNA

<213>  Artificial sequence

<220>

<223>  Artificial sequence description : PRIMER

<400>  27
gtgcctttaa acttttctcc                                                    20

<210>  28

<211>  20

<212>  DNA

<213>  Artificial sequence

<220>

<223>  Artificial sequence description : PRIMER

<400>  28
cgagaagggt taatattagg                                                    20

<210>  29

<211>  18

<212>  DNA

<213>  Artificial sequence

<220>

<223>  Artificial sequence description : PRIMER

<400>  29
gccgagtgtg actcggac                                                    18

<210>  30

<211>  22

<212>  DNA

<213>  Artificial sequence

<220>

<223>  Artificial sequence description : PRIMER

<400>  30
ggtctaacca catgctattt tc                                               22

<210>  31

<211>  20

<212>  DNA

<213>  Artificial sequence

<220>

<223>  Artificial sequence description : PRIMER

<400>  31
ctatgttaca tatacatcac                                                  20

<210>  32

<211>  23

<212>  DNA

<213>  Artificial sequence

<220>

<223>  Artificial sequence description : PRIMER

<400>  32
gtacaactta ttcaatgtag ttg                                              23

<210>  33

<211>  23

<212>  DNA

<213>  Artificial sequence



<220>

<223>  Artificial sequence description : PRIMER

<400>  33
gacattccag gtcctatttt tgg                                              23

<210>  34

<211>  20

<212>  DNA

<213>  Artificial sequence



<220>

<223>  Artificial sequence description : PRIMER

<400>  34
gcttggttct tcccacttac                                                  20

<210>  35

<211>  18

<212>  DNA

<213>  Artificial sequence



<220>

<223>  Artificial sequence description : PRIMER

<400>  35
ggtaggatgt ttttcatg                                                    18

<210>  36

<211>  20

<212>  DNA

<213>  Artificial sequence



<220>

<223>  Artificial sequence description : PRIMER

<400>  36
cctttgaaga ataaactcag                                                    20

<210>  37

<211>  19

<212>  DNA

<213>  Artificial sequence

<220>

<223>  Artificial sequence description : PRIMER

<400>  37
gacaaagctc catgttgtc                                                     19

<210>  38

<211>  20

<212>  DNA

<213>  Artificial sequence

<220>

<223>  Artificial sequence description : PRIMER

<400>  38
ctgtttctca gcccagactc                                                    20

<210>  39

<211>  19

<212>  DNA

<213>  Artificial sequence

<220>

<223>  Artificial sequence description : PRIMER

<400>  39
atccaagtgc ttaactgtg                                                     19

<210>  40

<211>  19

<212>  DNA

<213>  Artificial sequence

<220>

<223>  Artificial sequence description : PRIMER

<400>  40
gtatagcatt cactggatc                                                    19

<210>  41

<211>  19

<212>  DNA

<213>  Artificial sequence

<220>

<223>  Artificial sequence description : PRIMER

<400>  41
tacatccatt tggagacac                                                    19

<210>  42

<211>  17

<212>  DNA

<213>  Artificial sequence

<220>

<223>  Artificial sequence description : PRIMER

<400>  42
gcaaggctaa gaatttc                                                      17

<210>  43

<211>  19

<212>  DNA

<213>  Artificial sequence

<220>

<223>  Artificial sequence description : PRIMER

<400>  43
gccttttcta tgtctacag                                                    19

<210>  44

<211>  17

<212>  DNA

<213>  Artificial sequence

<220>

<223>  Artificial sequence description : PRIMER

<400>  44
agaagcagca gctgatg                                                    17

<210>  45

<211>  20

<212>  DNA

<213>  Artificial sequence

<220>

<223>  Artificial sequence description : PRIMER

<400>  45
ctcaagccac tatttatgag                                                 20

<210>  46

<211>  21

<212>  DNA

<213>  Artificial sequence

<220>

<223>  Artificial sequence description : PRIMER

<400>  46
agaatttgga agctccatta g                                               21

<210>  47

<211>  19

<212>  DNA

<213>  Artificial sequence

<220>

<223>  Artificial sequence description : PRIMER

<400>  47
caactcataa ctttgctac                                                  19

<210>  48

<211>  24

<212>  DNA

<213>  Artificial sequence

<220>

<223>  Artificial sequence description : PRIMER

<400>  48
catgcatatc gacataacaa taag                                              24

<210>  49

<211>  20

<212>  DNA

<213>  Artificial sequence

<220>

<223>  Artificial sequence description : PRIMER

<400>  49
ggtctcccct aaatttcctc                                                   20

<210>  50

<211>  21

<212>  DNA

<213>  Artificial sequence

<220>

<223>  Artificial sequence description : PRIMER

<400>  50
caagtgtggg tatgctacat g                                                 21

<210>  51

<211>  19

<212>  DNA

<213>  Artificial sequence

<220>

<223>  Artificial sequence description : PRIMER

<400>  51
gctggagcgc atgcatttg                                                    19

<210>  52

<211>  17

<212>  DNA

<213>  Artificial sequence



<220>

<223>  Artificial sequence description : PRIMER

<400>  52
gttgtagtgg gcacaaa                                                    17

<210>  53

<211>  22

<212>  DNA

<213>  Artificial sequence



<220>

<223>  Artificial sequence description : PRIMER

<400>  53
cttgaacaga ttatgccttt gg                                              22

<210>  54

<211>  20

<212>  DNA

<213>  Artificial sequence



<220>

<223>  Artificial sequence description : PRIMER

<400>  54
tcctagtcac atcaaaaagc                                                 20

<210>  55

<211>  17

<212>  DNA

<213>  Artificial sequence



<220>

<223>  Artificial sequence description : PRIMER

<400>  55
cttaaaccca ctcggcc                                                    17

<210>  56

<211>  23

<212>  DNA

<213>  Artificial sequence

<220>

<223>  Artificial sequence description : PRIMER

<400>  56
   cacaggagtc atttttttcc tac                                              23

<210>  57

<211>  20

<212>  DNA

<213>  Artificial sequence

<220>

<223>  Artificial sequence description : PRIMER

<400>  57
gactttcaaa catcatggag                                                 20

<210>  58

<211>  20

<212>  DNA

<213>  Artificial sequence

<220>

<223>  Artificial sequence description : PRIMER

<400>  58
cttatcctgt agctataatc                                                 20

<210>  59

<211>  19

<212>  DNA

<213>  Artificial sequence

<220>

<223>  Artificial sequence description : PRIMER

<400>  59
ctggcaccag aactatacc                                                    19

<210>  60

<211>  22

<212>  DNA

<213>  Artificial sequence

<220>

<223>  Artificial sequence description : PRIMER

<400>  60
attatgacaa tattggttgg gc                                                22

<210>  61

<211>  16

<212>  DNA

<213>  Artificial sequence

<220>

<223>  Artificial sequence description : PRIMER

<400>  61
gaagctgctg acaccc                                                       16

<210>  62

<211>  20

<212>  DNA

<213>  Artificial sequence

<220>

<223>  Artificial sequence description : PRIMER

<400>  62
gaagtgcctt gtccaagttg                                                   20

<210>  63

<211>  23

<212>  DNA

<213>  Artificial sequence

<220>

<223>  Artificial sequence description : PRIMER

<400>  63
aatagaactg tcaactgtta agc                                                23

<210>  64

<211>  17

<212>  DNA

<213>  Artificial sequence

<220>

<223>  Artificial sequence description : PRIMER

<400>  64
ttttccccct gtgcttg                                                       17

<210>  65

<211>  21

<212>  DNA

<213>  Artificial sequence

<220>

<223>  Artificial sequence description : PRIMER

<400>  65
gattagaaag gtaacatttt c                                                  21

<210>  66

<211>  18

<212>  DNA

<213>  Artificial sequence

<220>

<223>  Artificial sequence description : PRIMER

<400>  66
gggtcttcta aattgatc                                                      18